# EUROPÄISCHE PATENTSCHRIFT

(11) **EP 0 883 593 B1**
(45) Veröffentlichungstag und Bekanntmachung des Hinweises auf die Patenterteilung: **17.05.2000**
(21) Anmeldenummer: 97902358.7
(22) Anmeldetag: 11.02.1997
(51) Int. Cl.: C07C 45/38, C07C 47/04, B01J 27/18, B01J 37/34

(54) **VERFAHREN ZUR HERSTELLUNG VON FORMALDEHYD**
PROCESS FOR THE PREPARATION OF FORMALDEHYDE
PROCEDE DE FABRICATION DE FORMALDEHYDE

(30) Priorität: 13.02.1996 DE 19605213
(43) Veröffentlichungstag der Anmeldung: 16.12.1998
(73) Patentinhaber: BASF AKTIENGESELLSCHAFT, 67056 Ludwigshafen (DE)
(72) Erfinder: KNUTH, Bernhard, D-67246 Dirmstein (DE); DIERCKS, Rainer, D-67141 Neuhofen (DE)
(86) Internationale Anmeldenummer: EP9700615
(87) Internationale Veröffentlichungsnummer: WO9730015

(56) Entgegenhaltungen:
- EP-A- 0 104 666
- EP-A- 0 467 169
- CHEMICAL ABSTRACTS, vol. 105, no. 24, 15.Dezember 1986 Columbus, Ohio, US; abstract no. 210789, DENG J ET AL: "Silver-phosphorus catalyst and its application in making aldehyde by catalytic oxidation of alcohol" XP002028603 in der Anmeldung erwähnt & CN 85 100 530 A (FUDAN UNIVERSITY;PEOP. REP. CHINA) 10.März 1986

## Beschreibung

Die vorliegende Erfindung betrifft ein Verfahren zur Herstellung von Formaldehyd durch oxidative Dehydrierung von Methanol.

Verfahren zur Herstellung von Formaldehyd durch oxidative Dehydrierung von Methanol an einem aus Silberkristallen aufgebauten Katalysator-Festbett sind allgemein bekannt (vgl. Ullmanns Enzyklopädie der technischen Chemie, 3. Auflage, Urban und Schwarzenberg, München-Berlin, 1956, 7. Band, S. 660 bis 663).

Vorteilhafte Effekte, die bei der Verwendung von Phosphorverbindungen als Promotoren zur Oxidation von Methanol zu Formaldehyd in Gegenwart eines Silberkatalysators auftreten, sind weiterhin aus der CN-A-85100530, DE-A-4022603 und der JP-A-38227/83 bekannt.

Die EP-A-0 467 169 beschreibt die Herstellung von Formaldehyd durch oxidative Dehydrierung von Methanol an einem Katalysator-Festbett, das aus Schichten von Silberkristallen aufgebaut ist, die ein pulverförmiges phosphorhaltiges Salz als Promotor enthalten. Dieses phosphordotierte Silberkatalysator-Festbett wird hergestellt, indem man es vor Beginn der Formaldehydherstellung mit einer Phosphorverbindung in Kontakt bringt.

Diese Verfahren sind jedoch noch verbesserungsfähig, weil der Katalysator bei seinem Einsatz zur Formaldehydherstellung kontinuierlich an Aktivität verliert, was sich an einer abnehmenden Ausbeute zeigt. Nach einer relativ kurzen Zeit muß der Katalysator deshalb ausgetauscht und regeneriert werden, wozu der Formaldehydherstellungsprozeß unterbrochen werden muß. Da der Austausch des Katalystor-Festbetts und die mit der Wiederaufnahme des Formaldehydherstellungsprozesses verbundenen Maßnahmen zeit- und arbeitsintensiv sind, hängt die Wirtschaftlichkeit des Verfahrens davon ab, wie häufig man diesen Austausch vornehmen muß.

Die Aufgabe der vorliegenden Erfindung bestand deshalb darin, ein Verfahren zu entwickeln, bei dem der Zeitraum zwischen dem erforderlichen Austausch des Katalysators verlängert ist.

Demgemäß wurde ein Verfahren zur Herstellung von Formaldehyd durch oxidative Dehydrierung von Methanol gefunden, welches dadurch gekennzeichnet ist, daß man eine Gasmischung (i), enthaltend
a) 0,1 bis 50, bevorzugt 10 bis 40 Vol.-% Methanol,
b) 0,1 bis 30, bevorzugt 5 bis 20 Vol.-% Sauerstoff,
c) 0 bis 50, bevorzugt 1 bis 20 Vol.-% Stickstoffoxid und
d) 0 bis 60, bevorzugt 10 bis 50 Vol.-% Wasser
bei einer Temperatur von 150 bis 800°C durch ein phosphordotiertes Silberkatalysator-Festbett (a) leitet und man pro kg Methanol, das man in Form der Gasmischung (i), pro cm² der Querschnittsfläche des phosphordotierten Silberkatalysator-Festbetts (a) leitet, 0,01 bis 100 Gew.-ppm Phosphor, bezogen auf das phosphordotierte Silberkatalysator-Festbett, in Form einer feinverteilten Phosphorverbindung mit einer Schmelz- bzw. Zersetzungstemperatur von mehr als 500°C (Phosphorverbindung P), auf das phosphordotierte Silberkatalysator-Festbett (a) aufbringt.

Diese Angaben beziehen sich auf einen Druck von 1 bar.

Die Gasmischung enthält im allgemeinen 0,25 bis 0,60, bevorzugt 0,35 bis 0,50 Mol Sauerstoff pro Mol Methanol und 0,2 bis 3,0, bevorzugt 0,67 bis 1,75 Mol Wasser pro Mol Methanol und 0,9 bis 2,3, bevorzugt 1,3 bis 1,8 Mol Stickstoff pro Mol Methanol.

Für das erfindungsgemäße Verfahren eignen sich beispielsweise phosphordotierte Silberkatalysatoren, die erhältlich sind, indem man
I. Silberkristalle, die man durch elektrolytische Abscheidung von Silber aus einer wässerigen Silbersalzlösung erhält, zu einem Ausgangs-Silberkatalysator-Festbett (a) anordnet, und anschließend
II. das Ausgangs-Silberkatalysator-Festbett (a), mit 1 bis 20000 Gew.-ppm Phosphor, bezogen auf das Silber, in Form der Phosphorverbindung (P) in Kontakt bringt, bevor durch dieses bei einer Temperatur von 150 bis 800°C die Gasmischung (i) leitet.

Die in Schritt I beschriebene Herstellung der Silberkristalle ist allgemein bekannt (vgl. Ullmann's Enzyklopädie der technischen Chemie, 3. Auflage, Urban und Schwarzenberg, München-Berlin, 1956, 7. Band, S. 660 bis 663). Besonders gute Ergebnisse lassen sich mit den in der DE-A-2322757 beschriebenen Ausgangs-Katalysator-Festbetten erreichen.

Geeignete Silberkristalle erhält man insbesondere dann, wenn man die Elektrolyse nach dem in der deutschen Patentschrift 1166171 beschriebenen Verfahren durchführt.

Bevorzugt wird als Elektrolyt eine wäßrige Silbernitratlösung eingesetzt. Diese Silbernitratlösung hat im allgemeinen einen pH-Wert von 1 bis 4 und enthält 1 bis 5 Gew.-% Silber. Der pH-Wert wird günstigerweise mit Salpetersäure eingestellt.

Als Elektroden dienen die üblicherweise bei der Elektrolyse von Silber verwendeten Elektroden. Geeignete Anoden sind Säcke, in die das zu oxidierende Silber im allgemeinen als Granulat oder als Pulver eingefüllt worden ist. Als Kathoden kommen insbesondere Silberbleche in Betracht.

Die Elektrolyse wird günstigerweise bei Stromdichten von 80 bis 500 A/m² Kathodenfläche und Elektrolyttemperaturen von 10 bis 30°C durchgeführt.

Um diese Stromdichten zu erreichen, sind bei den meisten Elektrolysezellen Spannungen von 1 bis 15 Volt erforderlich.

Es empfiehlt sich, die an der Kathode gebildeten Silberkristalle fortwährend von der Kathode zu entfernen. Es werden im allgemeinen Silberkristalle mit einer Korngröße von 0,2 bis 5 mm erhalten.

Meistens reicht eine einmalige Elektrolyse aus, um brauchbare Silberkristalle zu erhalten.

Im allgemeinen ordnet man die Silberkristalle zu einem Ausgangs-Silberkatalysator-Festbett (a) an, das aus 1 bis 9 Schichten von Silberkristallen besteht und eine Gesamtschichtdicke von 1 bis 10 cm aufweist. Derartige Festbetten, die auch als "Kurzschichten" bezeichnet werden, sind allgemein bekannt (vgl. Ullmanns Enzyklopädie der technischen Chemie, 4. Auflage, Verlag Chemie - Weinheim-New York, Band 13, S. 539 bis 541).

Im Schritt II wird das Ausgangs-Silberkatalysator-Festbett (a) mit 1 bis 20000, bevorzugt 5 bis 5000 Gew.-ppm Phosphor, bezogen auf das Silber, in Form einer feinverteilten Phosphorverbindung mit einer Schmelz- bzw. Zersetzungstemperatur von mehr als 500°C (Phosphorverbindung P), in Kontakt gebracht.

Als Phosphorverbindungen (P) kommen phosphorhaltige Salze in Betracht. Beispiele hierfür sind die in der DE-A-4022603 genannten phosphorhaltigen Salze, z.B. anorganische Phosphate von Alkali-, Erdalkali-, und Schwermetallen wie Ag, Zn und Fe oder von Bor und Ammonium.

Bevorzugt sind Phosphate oder Pyrophosphate von Alkali oder Erdalkalimetallen, z.B. Na₄P₂O₇, Li₃PO₄, Mg₃(PO₄)₂, Ca₃(PO₄)₂.

Im allgemeinen geht man dabei so vor, daß man ein feinteiliges Pulver der Phosphorverbindung (P) auf das Silberkatalysator-Festbett aufstreut oder es mit einer Lösung der Phosphorverbindung (P) imprägniert und das Lösungsmittel verdunsten läßt.

Die Korngröße der als Pulver eingesetzten Phosphorverbindung (P) ist nicht kritisch, im allgemeinen beträgt sie ca. 1 mm bis 1 µm. Bei den Lösungen der Phosphorverbindungen (P) handelt es sich im allgemeinen um wässerige Lösungen, die 0,01 bis 50 Gew.-% der Phosphorverbindung (P) enthalten. Zur Imprägnierung des Silberkatalysator-Festbetts wird es mit einer dieser Lösungen getränkt oder besonders vorteilhaft die Lösungen auf das aktivierte Silberkatalysator-Festbett aufgesprüht und das Lösungsmittel anschließend verdunstet.

Die Menge an aufgesprühter oder aufgestreuter Phosphorverbindung (P) wird bevorzugt so gewählt, daß die Menge an Phosphor 0,01 bis 100, bevorzugt 0,05 bis 10 mg pro cm² der phosphordotierten Silberkatalysator-Festbett-Querschnittsfläche beträgt.

Die auf diese Weise hergestellten Silberkatalysator-Festbetten (a) entfalten beim Beginn der Durchleitung des Gasgemisches (i) im allgemeinen nicht von anfang an ihre volle katalytische Aktivität. Es hat sich deshalb als zweckmäßig erwiesen, die phosphordotierten Silberkatalysator-Festbetten (a) bei Beginn der Formaldehydherstellung zu aktivieren.

Die Aktivierung des Katalysators kann man beispielsweise vornehmen, indem man den Katalysator unmittelbar vor Beginn der Durchleitung des Gasgemisches (i) auf eine Temperatur von 300 bis 400°C vorheizt und/oder ein auf eine Temperatur von 100 bis 800, bevorzugt 200 bis 700°C vorgeheiztes Gasgemisch (i), durch das Festbett leitet. Die Menge an Methanol in Form der Gasmischung (i), die man bei Beginn der Aktivierungsphase, die üblicherweise 0,1 bis 100 Stunden dauert, pro Stunde und pro cm² Querschnittsfläche des phosphordotierten Silberkatalysator-Festbetts (a) durch dieses leitet, beträgt 0,001 bis 1 kg. Die Menge an Gasmischung (i), die man pro Zeiteinheit durch das phosphordotierte Silberkatalysator-Festbett (a) leitet, wird während der Aktivierungsphase kontinuierlich auf den Wert der Endbelastung erhöht, der im allgemeinen 0,1 bis 1 kg beträgt. Im allgemeinen erübrigt sich das Vorheizen der Gasmischung (i) spätestens nach Ende der Aktivierungsphase, da sich das Festbett durch die freiwerdende Reaktionswärme auf die erforderliche Temperatur aufheizt.

Damit sich Aktivierungsphase und die Herstellung von Formaldehyd nach dem erfindungsgemäßen Verfahren nahtlos aneinander anschließen können, nimmt man die Aktivierung des Ausgangs-Katalysator-Festbetts (a) zweckmäßigerweise in einem Festbettreaktor vor, wie man ihn üblicherweise für die Herstellung von Formaldehyd durch oxidative Dehydrogenierung von Methanol verwendet und leitet durch diesen kontinuierlich die Gasmischung (i). Vorzugsweise steht der Reaktor dabei senkrecht und die Gasmischung (i) wird von oben nach unten durch den Reaktor geleitet. Solche Reaktoren bzw. Verfahren sind beispielsweise in der EP-A-467 169, der DE-A-2444586 und der EP-A-0150436 beschrieben.

Günstigerweise wählt man die Querschnittsfläche des Reaktors und des Ausgangs-Silberkatalysator-Festbetts (b) gleich und ordnet es so in dem Reaktor an, daß die Schichten der Silberkristalle senkrecht zur Strömungsrichtung der Gasmischung (i) liegen.

Die phosphordotierten Silberkatalysator-Festbetten (a) haben am Ende der Aktivierungsphase ihr Aktivitätsmaximum und verlieren bei Ihrer Verwendung im erfindungsgemäßen Verfahren langsam stetig an Aktivität, was an sinkender Ausbeute an Formaldehyd bemerkbar ist.

Dieser Aktivitätsverlust der phosphordotierten Silberkatalysator-Festbetten (a) läßt sich teilweise vermeiden, wenn man pro kg Methanol in Form der Gasmischung (i), das man, bezogen auf 1 cm² der Querschnittsfläche des phosphordotierten Silberkatalysator-Festbetts (a), durch dieses leitet, entweder kontinuierlich oder diskontinuierlich (in jeweils einer Portion nach Einleitung einer definierten Menge an Gasmischung (i)) 0,01 bis 100 Gew.-ppm Phosphor, bezogen auf das phosphordotierte Silberkatalysator-Festbett, in Form der Phosphorverbindung (P) auf dieses aufbringt, bevorzugt ohne die Einleitung der Gasmischung (i) zu unterbrechen. Bei kontinuierlichem Aufbringen kann der Aktivitätsverlust verlangsamt, bei der schrittweisen, diskontinuierlichen Aufbringung teilweise rückgängig gemacht werden.

Wird das nachträgliche Aufbringen der Phosphorverbindung (P) diskontinuierlich vorgenommen, so werden die Intervalle zwischen dem nachträglichen Aufbringen der Phosphorverbindung (P) auf das phosphordotierte Silberkatalysator-Festbett so lang gewählt, daß in dieser Zeit nicht mehr als 500, bevorzugt 1 bis 5 kg Methanol in Form der Gasmischung (M), bezogen auf 1 cm² der Querschnittsfläche des phosphordotierten Silberkatalysator-Festbetts, durch dieses geleitet werden, da ansonsten zwischenzeitlich die Ausbeute zu stark absinken würde.

Das Herstellungsverfahren von Formaldehyd durch oxidative Dehydrogenierung von Methanol unter Verwendung des erfindungsgemäßen Katalysator-Festbetts wird im übrigen in an sich bekannter Weise durchgeführt, indem man das Gasgemisch (i) bei Temperaturen von etwa 500 bis 750°C, insbesondere 600 bis 710°C durch das phosphordotierte Silberkatalysator-Festbett leitet. Das Verfahren wird im allgemeinen bei einem Druck von 0,5 bis 2 bar, vorzugsweise von 0,8 bis 1,8 bar kontinuierlich durchgeführt. Dabei ist es vorteilhaft, die die Katalysatorzone verlassenden Reaktionsgase innerhalb kurzer Zeit abzukühlen, z.B. auf Temperaturen von 50 bis 350°C.

Vorzugsweise befindet sich das phosphordotierte Silberkatalysator-Festbett in einem senkrecht stehenden Reaktor und die Gasmischung (i) wird von oben nach unten durch den Reaktor geleitet. Günstigerweise wählt man die Querschnittsfläche des Reaktors und des Ausgangs-Silberkatalysator-Festbetts (b) gleich und ordnet es so in dem Reaktor an, daß die Schichten der Silberkristalle senkrecht zur Strömungsrichtung der Gasmischung (i) liegen.

Das abgekühlte Gasgemisch wird dann zweckmäßigerweise einem Absorptionsturm zugeführt, in welchem der Formaldehyd mit Wasser oder einer wässerigen Formaldehyd-Harnstoff-Lösung aus dem Gasgemisch gewaschen wird.

Spezielle vorteilhafte Varianten des allgemein bekannten Verfahrens zur Herstellung von Formaldehyd, die auch beim erfindungsgemäßen Verfahren angewendet werden können, sind in der DE-A-2444586, der DE-A-2451990, der EP-A-0083427 und der EP-A-0150436, empfohlen.

Das erfindungsgemäße Verfahren zeichnet sich insbesondere dadurch aus, daß sich mit ihm Formaldehyd besonders wirtschaftlich herstellen läßt, weil bei ihm die Ausbeute und die Selektivität bei der oxidativen Dehydrierung von Methanol über einen langen Zeitraum hinweg besonders hoch ist.

### Beispiel 1

In einem senkrecht stehenden Versuchsreaktor mit einem Innendurchmesser von 15 cm wurde ein dreischichtiges Ausgangs-Silberkatalysator-Festbett gleichen Durchmessers mit einer Gesamtschichtdicke von 2 cm angebracht. Die untere Schicht bestand aus 1000 g Silberkristallen der Korngröße 1 bis 2,5 mm, die mittlere Schicht aus 65 g Silberkristallen der Korngröße von 0,75 bis 1 mm und die obere Schicht aus 185 g Silberkristallen der Korngröße 0,2 bis 0,75 mm.

Auf die Oberfläche des Ausgangs-Silberkatalysator-Festbetts wurde Phosphor in Form von pulverförmigem Na₄P₂O₇ in einer Menge von 1,3 mg Phosphor (berechnet als elementarer Phosphor) pro cm² Festbettquerschnittsfläche aufgestreut. Das so hergestellte phophordotierte Silberkatalysator-Festbett wurde auf 360°C aufgeheizt. Anschließend wurde zur Aktivierung des Katalysators eine Gasmischung bestehend aus Methanol, Luft und Wasser durch den Katalysator geleitet. Die Menge wurde während der 28-stündigen Aktivierungsperiode auf pro Stunde 32 kg Methanol, 21,4 kg Wasser und 54 kg Luft erhöht (Endbelastung). Am Ende der Aktivierungsperiode betrug die Temperatur in dem Festbett 700°C. Dieser Mengenstrom wurde während des gesamten Versuchsdauer konstant gehalten.

Während des kontinuierlichen Betriebs des Reaktors wurden nach den in Tabelle 1 angegebenen Betriebszeiten (Betriebstag 0 bedeutet der Zeitpunkt unmittelbar nach der Aktivierung des Katalysators) weitere Phosphormengen in Form von pulverförmigem Na₄P₂O₇ auf das phophordotierte Silberkatalysator-Festbett aufgebracht. Die jeweiligen Mengen sind in Tabelle 1 angeben (kumulierte Mengen).

**Tabelle 1**

| Betriebstage | Phosphormenge [g/cm²] | Ausbeute [%] |
|---|---|---|
| 0 | 1,3 | 90,1 |
| 4 | 1,3 | 89,7 |
| 6 | 1,42 | 90,1 |
| 9 | 1,42 | 89,6 |
| 11 | 1,61 | 90,0 |
| 16 | 1,61 | 89,3 |
| 17 | 1,70 | 89,9 |

## Patentansprüche

1. Verfahren zur Herstellung von Formaldehyd durch oxidative Dehydrierung von Methanol, dadurch gekennzeichnet, daß man eine Gasmischung (i), enthaltend
a) 0,1 bis 50 Vol.-% Methanol,
b) 0,1 bis 30 Vol.-% Sauerstoff,
c) 0 bis 50 Vol.-% Stickstoffoxid und
d) 0 bis 60 Vol.-% Wasser
bei einer Temperatur von 150 bis 800°C durch ein phosphordotiertes Silberkatalysator-Festbett (a) leitet und man pro kg Methanol, das man in Form der Gasmischung (i), pro cm² der Querschnittsfläche des phosphordotierten Silberkatalysator-Festbetts (a) leitet, 0,01 bis 100 Gew.-ppm Phosphor, bezogen auf das phosphordotierte Silberkatalysator-Festbett, in Form einer feinverteilten Phosphorverbindung mit einer Schmelz- bzw. Zersetzungstemperatur von mehr als 500°C (Phosphorverbindung P), auf das phosphordotierte Silberkatalysator-Festbett (a) aufbringt.

2. Verfahren nach Anspruch 1, dadurch gekennzeichnet, daß man ein phosphordotiertes Silberkatalysator-Festbett (a) einsetzt, welches erhältlich ist, indem man
I. Silberkristalle, die man durch elektrolytische Abscheidung von Silber aus einer wässerigen Silbersalzlösung erhält, zu einem Ausgangs-Silberkatalysator-Festbett (a) anordnet, und anschließend
II. das Ausgangs-Silberkatalysator-Festbett (a) mit 1 bis 20000 Gew.-ppm, Phosphor, bezogen auf das Silber, in Form der Phosphorverbindung (P) in Kontakt bringt, bevor man durch dieses bei einer Temperatur von 150 bis 800°C die Gasmischung (i) leitet.

3. Verfahren nach Anspruch 1 oder 2, dadurch gekennzeichnet, daß man ein phosphordotiertes Silberkatalysator-Festbett (a), das aus einer oder mehreren Schichten von Silberkristallen, deren längster mittlerer Durchmesser 0,2 bis 10 mm beträgt, aufgebaut ist, und wobei die Gesamtschichtdicke 1 bis 10 cm beträgt, einsetzt.

4. Verfahren nach den Ansprüchen 1 bis 3, dadurch gekennzeichnet, daß man als Phosphorverbindung (P) Phosphate oder Pyrophosphate von Alkali oder Erdalkalimetallen einsetzt.

## Claims

1. A process for preparing formaldehyde by oxidative dehydrogenation of methanol, which comprises passing a gas mixture (i) comprising
a) from 0.1 to 50 % by volume of methanol,
b) from 0.1 to 30 % by volume of oxygen,
c) from 0 to 50 % by volume of nitrogen oxide and
d) from 0 to 60 % by volume of water
at from 150 to 800°C through a phosphorus-doped silver catalyst fixed bed (a) and applying from 0.01 to 100 ppm by weight of phosphorus, based on the phosphorus-doped silver catalyst fixed bed, in the form of a finely divided phosphorus compound having a melting point or decomposition temperature of more than 500°C (phosphorus compound P) to the phosphorus-doped silver catalyst fixed bed (a) per kg of methanol which is passed through in the form of the gas mixture (i) per cm² of the cross-sectional area of the phosphorus-doped silver catalyst fixed bed (a).

2. A process as claimed in claim 1, wherein the phosphorus-doped silver catalyst fixed bed (a) used is obtainable by
I. arranging silver crystals which are obtained by electrolytic deposition of silver from an aqueous silver salt solution to form a starting silver catalyst fixed bed (a), and subsequently
II. bringing the starting silver catalyst fixed bed (a) into contact with from 1 to 20,000 ppm by weight of phosphorus, based on the silver, in the form of the phosphorus compound (P) before the gas mixture (i) is passed at from 150 to 800°C through the fixed bed.

3. A process as claimed in claim 1 or 2, wherein the phosphorus-doped silver catalyst fixed bed (a) used is made up of one or more layers of silver crystals whose longest mean diameter is from 0.2 to 10 mm and the total bed thickness is from 1 to 10 cm.

4. A process as claimed in any of claims 1 to 3, wherein the phosphorus compound (P) used is a phosphate or pyrophosphate of an alkali metal or alkaline earth metal.

## Revendications

1. Procédé de préparation de formaldéhyde par déshydrogénation oxydante de méthanol, caractérisé en ce que l'on conduit un mélange de gaz (i), contenant
a) 0,1 à 50 % en volume de méthanol,
b) 0,1 à 30 % en volume d'oxygène,
c) 0 à 50 % en volume d'oxyde d'azote et
d) 0 à 60 % en volume d'eau,
à une température de 150 à 800°C à travers un lit fixe de catalyseur à l'argent dopé au phosphore (a) et en ce que l'on dépose, par kg de méthanol introduit sous forme du mélange de gaz (i), et par cm² de la surface de la section du lit fixe de catalyseur à l'argent dopé au phosphore (a), 0,01 à 100 ppm en poids de phosphore, par rapport au lit fixe de catalyseur à l'argent dopé au phosphore, sous forme d'un composé du phosphore finement divisé avec une température de fusion ou de décomposition de plus de 500°C (composé du phosphore P), sur le lit fixe de catalyseur à l'argent dopé au phosphore (a).

2. Procédé selon la revendication 1, caractérisé en ce que l'on utilise un lit fixe de catalyseur à l'argent dopé au phosphore (a) pouvant être obtenu par
I. agencement de cristaux d'argent obtenus par séparation électrolytique d'argent à partir d'une solution aqueuse de sel d'argent, en un lit fixe de catalyseur à l'argent de départ (a) et ensuite
II. mise en contact du lit fixe de catalyseur à l'argent de départ (a) avec 1 à 20000 ppm en poids de phosphore, par rapport à l'argent, sous forme du composé du phosphore (P), avant que l'on conduise le mélange de gaz (i) à travers lui à une température de 150 à 800°C.

3. Procédé selon la revendication 1 ou 2, caractérisé en ce que l'on utilise un lit fixe de catalyseur à l'argent dopé au phosphore (a) construit à partir d'une ou plusieurs couches de cristaux d'argent dont le plus grand diamètre moyen maximal est de 0,2 à 10 mm, et où l'épaisseur totale des couches vaut de 1 à 10 cm.

4. Procédé selon l'une quelconque des revendications 1 à 3, caractérisé en ce que l'on utilise en tant que composé du phosphore (P) des phosphates ou des pyrophosphates de métaux alcalins ou alcalino-terreux.
